# EUROPEAN PATENT APPLICATION

(11) **EP 4 643 775 A2**
(43) Date of publication of application: **05.11.2025**
(21) Application number: 23912940.6
(22) Date of filing: 27.12.2023
(51) Int. Cl.: A61B 5/145, A61B 5/1486, G01N 27/30, G01N 27/327, G01N 27/407, G01N 33/48

(54) **COMPLEX SENSOR CAPABLE OF MEASURING TWO OR MORE ANALYTES**

(30) Priority: 29.12.2022 KR 20220189323; 24.11.2023 KR 20230165283
(71) Applicant: I-SENS, INC., Seoul 06646 (KR)
(72) Inventor: NAM, Hak Hyun, Seoul 06646 (KR); CHOI, Won Seok, Seoul 06646 (KR); CHOI, Hyun Ho, Seoul 06646 (KR); CHO, Hee Gyung, Seoul 06646 (KR); KU, Ye Sol, Seoul 06646 (KR)
(74) Representative: Westphal, Mussgnug & Partner Patentanwälte mbB
(86) International application number: PCT/KR2023/021751
(87) International publication number: WO 2024/144266

(57) **Abstract**

According to various embodiments, a composite sensor may be provided comprising: a first sensor including a first substrate comprising a first body and a first probe, a first electrode, and a first sensing layer disposed on the first probe; a second sensor including a second substrate comprising a second body and a second probe, a second electrode, and a second sensing layer disposed on the second probe; and a counter electrode disposed between the first sensor and the second sensor; wherein, when the first sensing layer contacts an analyte in a body, the counter electrode is configured to cause a first potential difference with the first electrode based on a first component included in the analyte, and when the second sensing layer contacts the analyte, the counter electrode is configured to cause a second potential difference with the second electrode based on a second component included in the analyte.

## Description

### [Technical Field]

The present invention relates to a composite sensor capable of measuring two or more analytes, and more particularly to a composite sensor capable of detecting two or more analytes contained in a body.

### [Background Art]

Existing diabetic patients periodically analyzed blood collected from a part of the body to measure glucose concentration in the body. However, the existing method not only caused pain to patients, but also had the inconvenience of having to periodically perform the process of collecting blood.

Accordingly, in the modern medical industry, demand for continuous glucose monitoring system (CGMS) technology that can continuously measure blood glucose in the body while being attached to a user's body for a certain period is increasing. Continuous glucose monitoring technology is provided by measuring glucose concentration using a sensor that can contact interstitial fluid in the body.

This research was supported by a national research and development project conducted by the Korea Ministry of Science and ICT and the Pan-Ministry Full-Cycle Medical Device Research and Development Foundation, and was conducted through the research project 'Development of Ultra-small Continuous Glucose Monitor for Building Smart Healthcare System' (Project unique number: 1711174351, Project number: 00141116) as part of the Pan-Ministry Full-Cycle Medical Device Research and Development Project (R&D).

This research was supported by a national research and development project conducted by the Ministry of Health and Welfare of Korea and the Korea Health Industry Development Institute, and was conducted through the research project 'Development of Ultra-small Continuous Glucose Monitor Linked to Weekly-use Patch-type Insulin Pump' (Project unique number: 1465040368, Project number.: HI23C0048000123) as part of the Drug Delivery Treatment Technology Development Project.

### [Detailed Description of the Invention]

### [Technical Problem]

Along with glucose, ketone is also one of the indicators that can represent the current state of diabetic patients.

The problem to be solved according to embodiments of the present specification aims to more accurately measure the blood glucose state of diabetic patients by providing a sensor of a new structure capable of measuring both glucose and ketone.

The problems of the present invention are not limited to the problems mentioned above, and other problems not mentioned will be clearly understood by those skilled in the art from the following description.

### [Technical Solution]

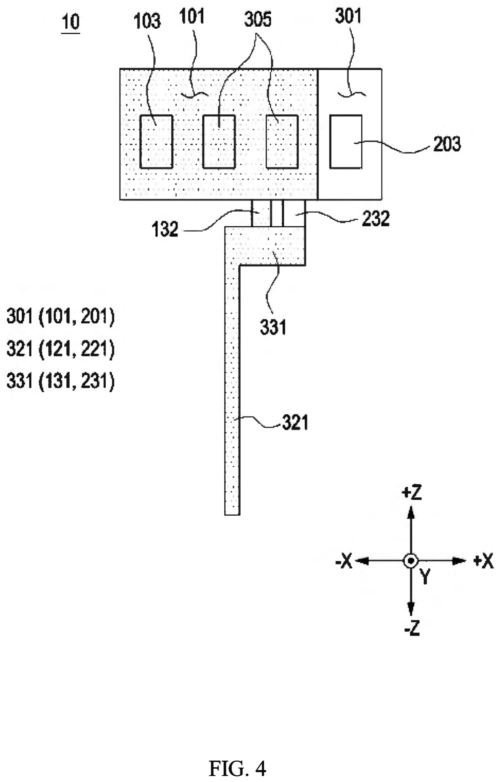

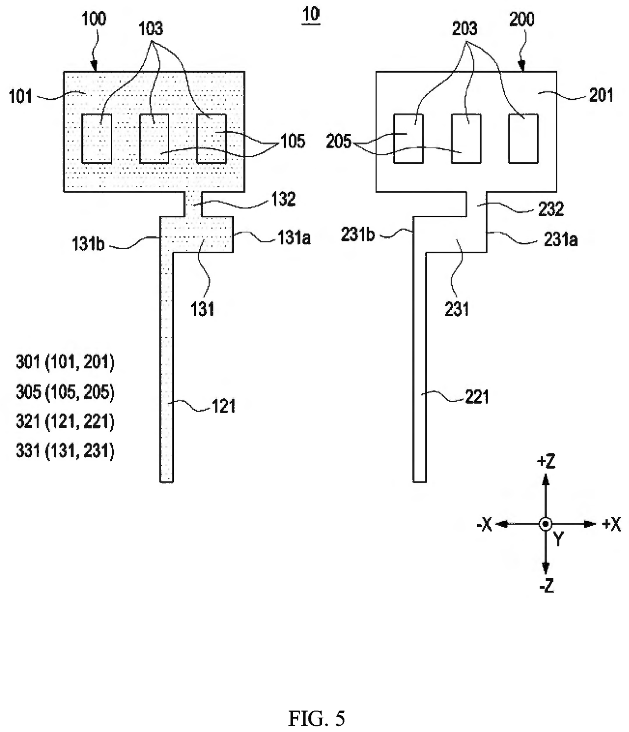

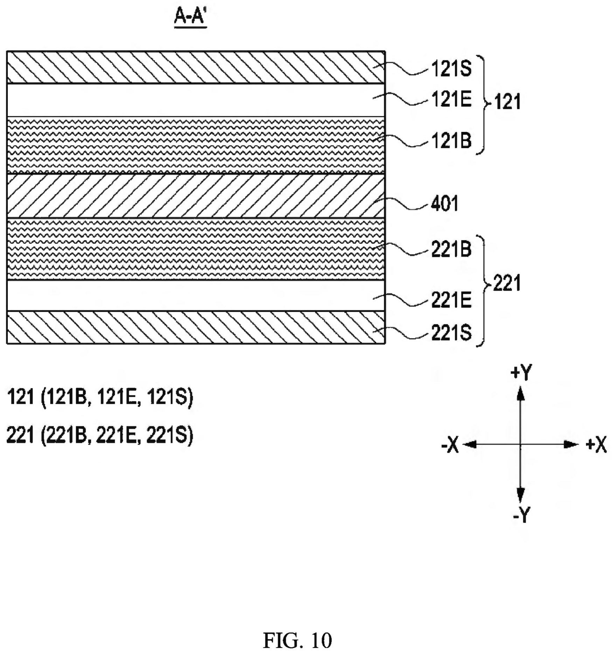

According to various embodiments, a composite sensor may be provided comprising: a first sensor (e.g., first sensor (100) of FIG. 5) including a first substrate (e.g., first substrate (121B) of FIG. 10) comprising a first body (e.g., first body (101) of FIG. 4) and a first probe (e.g., first probe (121) of FIG. 4) extending from the first body, a first electrode (e.g., first electrode (121E) of FIG. 10) disposed on a surface located in a first direction of the first substrate, and a first sensing layer (e.g., first sensing layer (121S) of FIG. 10) electrically connected to the first electrode and disposed on the first probe; a second sensor (e.g., second sensor (200) of FIG. 5) including a second substrate (e.g., second substrate (221B) of FIG. 10) comprising a second body (e.g., second body (201) of FIG. 4) and a second probe (e.g., second probe (221) of FIG. 5) disposed in a region extending from the second body, a second electrode (e.g., second electrode (221E) of FIG. 10) disposed on a surface located in a second direction opposite to the first direction of the second substrate, and a second sensing layer (e.g., second sensing layer (221S) of FIG. 10) electrically connected to the second electrode and disposed on the second probe; and a counter electrode (e.g., counter electrode (931) of FIG. 10) disposed between the first sensor and the second sensor; wherein, when the first sensing layer contacts an analyte in a body, the counter electrode is configured to cause a first potential difference with the first electrode based on a first component included in the analyte, and when the second sensing layer contacts the analyte, the counter electrode is configured to cause a second potential difference with the second electrode based on a second component included in the analyte.

### [Advantageous Effects]

The present invention can provide a sensor capable of measuring both glucose and ketone through one assembly.

The effects according to the present invention are not limited by the contents exemplified above, and more various effects are included in the present specification.

### [Brief Description of Drawings]

FIG. 1 is an implementation example of a continuous glucose monitoring system using a sensor member according to various embodiments.
FIG. 2 is an implementation example of an applicator for applying a sensor member to a user according to various embodiments.
FIG. 3 is an implementation example of a body attachable unit of a continuous glucose monitoring system using a sensor member according to various embodiments.
FIG. 4 is a diagram showing a sensor member as a whole according to various embodiments. FIG. 5 is a diagram showing a first sensor and a second sensor.
FIG. 6 is a diagram showing a sensor member from another angle according to various embodiments.
FIG. 7 is a diagram showing a bent sensor member according to various embodiments.
FIG. 8 is a diagram showing area A of FIG. 6.
FIG. 9 is a cross-sectional view of section B-B' of FIG. 6.
FIG. 10 is a cross-sectional view of section A-A' of FIG. 8.
FIG. 11 is a cross-sectional view of section C-C' of FIG. 8.
FIG. 12 is a diagram showing a sensor member according to another embodiment.
FIG. 13 is a diagram showing a first surface of a sensor base according to various embodiments.
FIG. 14 is a diagram showing a second surface of a sensor base according to various embodiments.
FIG. 15 is a diagram showing a folded sensor base according to various embodiments.
FIG. 16 is a flowchart showing a manufacturing process of a sensor member according to various embodiments.
FIG. 17 is a diagram showing a via hole structure according to various embodiments. 1.

### [Best Mode for Carrying Out the Invention]

The above-mentioned objects, features and advantages of the present invention will become more apparent through the following detailed description related to the accompanying drawings. However, since the present invention can have various modifications and various embodiments, specific embodiments will be illustrated in the drawings and described in detail below.

In the drawings, the thickness of layers and regions is exaggerated for clarity, and when an element or layer is referred to as being "on" another element or layer, it includes not only directly on the other element or layer but also cases where other layers or other elements are interposed in between. Throughout the specification, the same reference numerals indicate the same elements in principle. In addition, elements having the same function within the scope of the same concept appearing in the drawings of each embodiment are described using the same reference numerals.

When it is determined that a detailed description of known functions or configurations related to the present invention may unnecessarily obscure the gist of the present invention, the detailed description thereof is omitted. In addition, numbers (for example, first, second, etc.) used in the description process of the present specification are merely identification symbols for distinguishing one element from other elements.

In addition, the suffixes "region", "portion" and "part" for elements used in the following description are given or used interchangeably only considering the ease of writing the specification, and do not have meanings or roles that are distinct from each other by themselves.

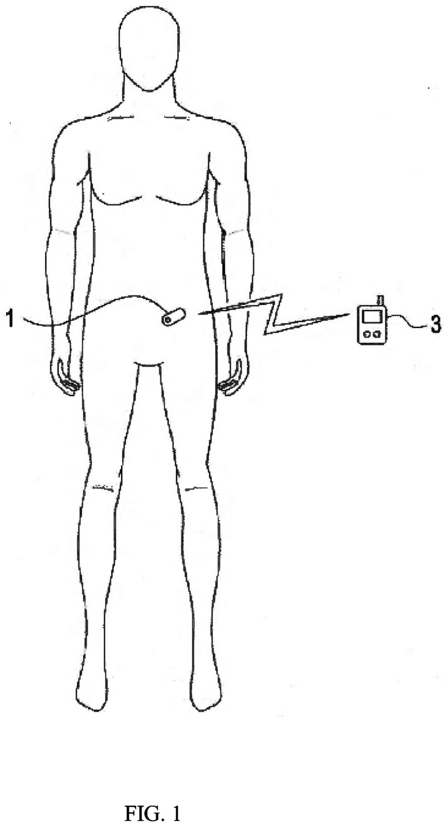

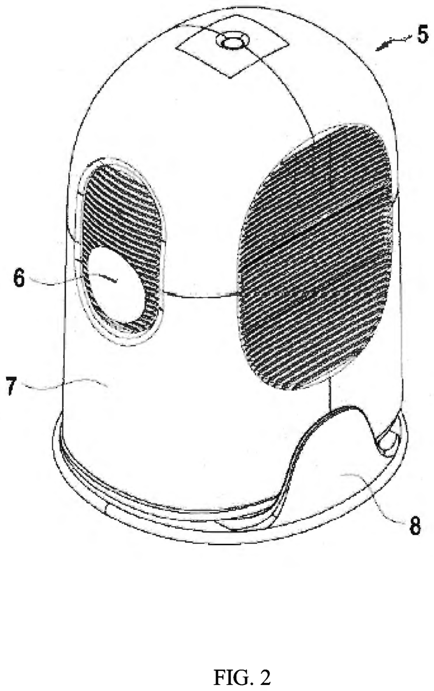

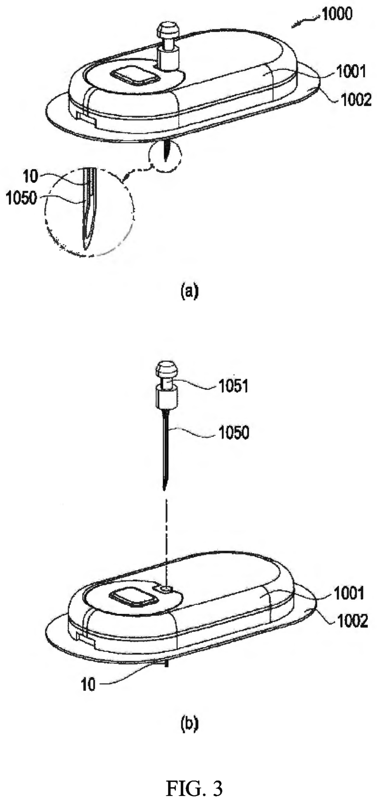

FIG. 1 is an implementation example of a continuous glucose monitoring system using a sensor member according to various embodiments. FIG. 2 is an implementation example of an applicator for applying a sensor member to a user according to various embodiments. FIG. 3 is an implementation example of a body attachable unit of a continuous glucose monitoring system using a sensor member according to various embodiments.

According to various embodiments, the continuous glucose monitoring device is configured to attach a body attachable unit (1000) having a sensor member (10) inserted into the body for continuous glucose measurement to the body through an applicator (5), and operates the applicator (5) to insert and attach the body attachable unit (1000) to the body to continuously measure blood glucose from the body, and blood glucose measurement information periodically measured through the body attachable unit (1000) is configured to be transmitted to a separate terminal (3) and output.

The body attachable unit (1000) is assembled inside the applicator (5) to be manufactured as one unit product, and is configured with a very simple structure in which the usage method minimizes additional work by the user when using the continuous glucose monitoring device.

The body attachable unit (1000) is formed to be attachable to the body so as to extract body fluid and periodically measure blood glucose, and is formed to transmit blood glucose measurement results to external devices such as an external terminal (3). In this body attachable unit (1000), a sensor member (10) having one end inserted into the body and a wireless communication chip disposed inside to enable wireless communication with the external terminal (3) are provided, so that it can be manufactured in a usable form without the need to additionally couple a separate transmitter.

The applicator (5) is formed so that the body attachable unit (1000) is coupled and fixed inside, and operates to discharge the body attachable unit (1000) to the outside by a user's pressing operation on the button (6).

At this time, the body attachable unit (1000) is assembled and manufactured in a state inserted inside the applicator (5), and is configured to move in the external discharge direction and be attached to the body according to the operation of the applicator (5) by the user's manipulation.

That is, the sensor applicator assembly according to various embodiments is assembled and manufactured such that the body attachable unit (1000) is attached to the skin only by the operation of the applicator (5) in a state where the body attachable unit (1000) is inserted inside the applicator (5) at the manufacturing stage, and is supplied to the user in this state, so that the user can attach the body attachable unit (1000) to the skin simply by operating the applicator (5) without any additional work for attaching the body attachable unit (1000) to the skin. In particular, since the body attachable unit (1000) is provided with a separate wireless communication chip, it is not necessary to couple a separate transmitter, so it can be used more conveniently.

In various embodiments, by manufacturing and distributing the body attachable unit (1000) in a state inserted into the applicator (5) at the manufacturing stage, processes such as the user peeling off the body attachable unit (1000) and inserting it into the applicator (5) are omitted, and the body attachable unit (1000) can be attached to the skin simply by operating the applicator (5), so usability is dramatically improved, and in particular, contamination of the body attachable unit (1000) can be prevented, thereby improving blood glucose measurement accuracy.

A separate protective cap (8) may be detachably coupled to the applicator (5) so as to block external exposure in a state where the body attachable unit (1000) is inserted inside the applicator (5), and the user may be configured to operate the applicator (5) only after removing the protective cap (8) to externally discharge the body attachable unit (1000) toward the side where the protective cap (8) was removed and attach it to the body.

At this time, an adhesive tape (1002) is attached to the body contact surface of the body attachable unit (1000) so that the body attachable unit (1000) can be attached to the body, and a release paper (not shown) is attached to the body contact surface of the adhesive tape (1002) for protection of the adhesive tape (1002), wherein the release paper of the adhesive tape (1002) may be formed to be separated and removed from the adhesive tape (1002) in the process of separating the protective cap (8) from the applicator (5). However, this is exemplary, and various implementation modifications are possible.

For example, the release paper of the adhesive tape (1002) may be configured such that one side is adhered to the protective cap (8), and therefore, when the user separates the protective cap (8) from the applicator (5), it can be separated and removed from the adhesive tape (1002) together with the protective cap (8). Accordingly, when the user separates the protective cap (8), the release paper of the adhesive tape (1002) is separated and removed, so in this state, the applicator (5) can be operated to attach the body attachable unit (1000) to the body.

In addition, the applicator (5) may be formed to couple and fix the body attachable unit (1000) in a state where the body attachable unit (1000) is inserted inside, and to release the coupled and fixed state with respect to the body attachable unit (1000) in a state where the body attachable unit (1000) has moved for external discharge. Therefore, in a state where the body attachable unit (1000) is inserted and assembled inside the applicator (5), the body attachable unit (1000) is maintained in a fixed state, and when the applicator (5) is operated to externally discharge the body attachable unit (1000) to be attached to the skin, the coupled and fixed state between the applicator (5) and the body attachable unit (1000) is released, so when the applicator (5) is separated in this state, it is separated from the body attachable unit (1000), and only the body attachable unit (1000) remains attached to the skin.

Meanwhile, the body attachable unit (1000) according to various embodiments may be formed such that the sensor member (10) and the wireless communication chip start operating through separate switch means operated by the user. That is, after inserting and attaching the body attachable unit (1000) to the body through the applicator (5), the user can cause the body attachable unit (1000) to start operating through switch means provided in the body attachable unit (1000), and from this operation start time, the sensor member (10) and the wireless communication chip operate to measure blood glucose of the body and transmit measurement results to the external terminal (3). At this time, the switch means operated by the user may be configured in various ways.

In addition, in the body attachable unit (1000), the sensor member (10) is disposed inside the housing (1001), and one end of the sensor member (10) is formed to protrude outward from the bottom surface of the housing (1001) so as to be inserted and attached to the body. The sensor member (10) is composed of a sensor probe inserted into the body and a sensor body disposed inside the housing (1001), wherein the sensor probe and the sensor body are bent to form one end and the other end of the sensor member (10), respectively.

At this time, a separate insertion guide needle (1050) may be detachably coupled to the housing (1001) so that the body insertion process of the sensor member (10) is performed smoothly. The insertion guide needle (1050) is configured to surround one end of the sensor member (10) and be inserted into the body together with the sensor member (10) so that one end of the sensor member (10) is stably inserted into the body.

This insertion guide needle (1050) is detachably mounted in a direction penetrating through the housing (1001) of the body attachable unit (1000) up and down as shown in FIG. 3, is formed to surround the outside of the sensor member (10), and has a needle head (1051) formed at the upper end. When the body attachable unit (1000) is moved in the external discharge direction by the applicator (5), this insertion guide needle (1050) is inserted into the body before the sensor member (10) and assists the sensor member (10) to be stably inserted into the skin. The insertion guide needle (1050) is coupled with a needle withdrawal body disposed inside the applicator (5) through the needle head (1051), and is formed to be withdrawn and removed from the body by the needle withdrawal body of the applicator (5) after the body attachable unit (1000) is inserted and attached to the body by the operation of the applicator (5).

After the insertion guide needle (1050) is withdrawn and removed from the body, the body attachable unit (1000) is maintained in a state inserted and attached to the body, and the sensor member (10) of the body attachable unit (1000) measures blood glucose information from body fluid in a state where one end is inserted into the skin. For measuring this blood glucose information, a plurality of electrode layers are formed on the sensor member (10), and each electrode layer is formed to be connected to electrical contacts of a separate external electronic device.

In the above and below description of the present invention, the term Continuous Glucose Monitoring System (CGMS) is used for convenience of description, but the spirit of the present invention is not limited thereto. According to various embodiments of the present invention, the continuous glucose monitoring system can acquire information about not only glucose in the body but also other analytes, and provide analysis results based thereon. In other words, the continuous glucose monitoring system of the present invention can acquire information about two or more analytes and analyze them.

Hereinafter, a sensor member for acquiring information about two or more analytes in the body will be described.

FIGS. 4 to 11 are drawings showing a sensor member (10) according to a first embodiment.

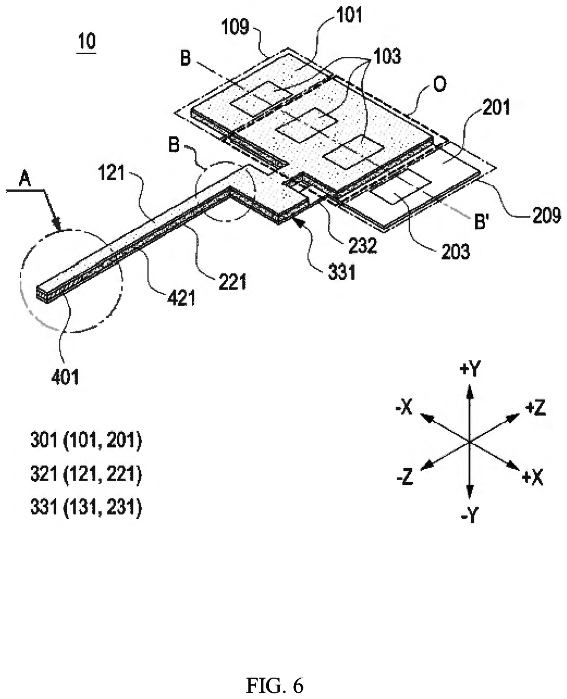

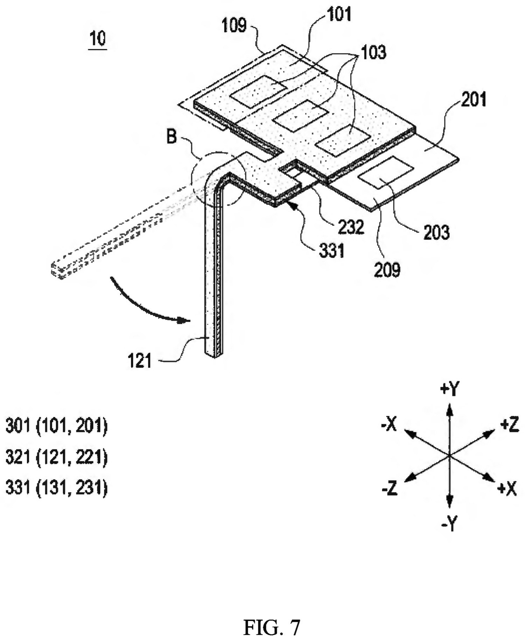

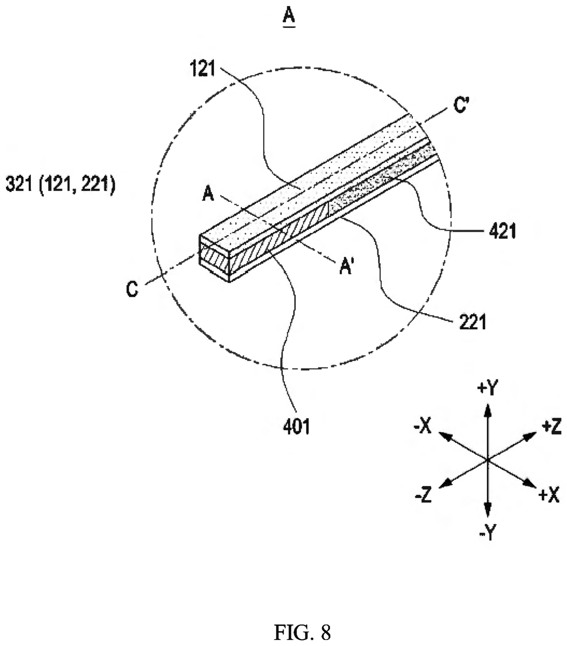

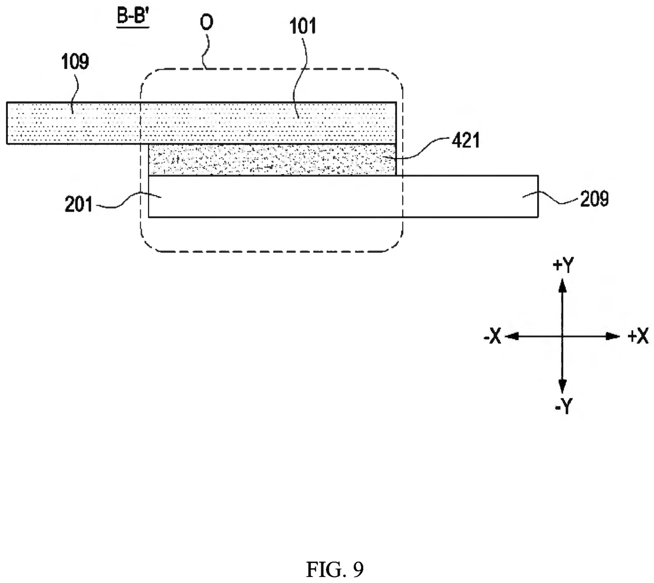

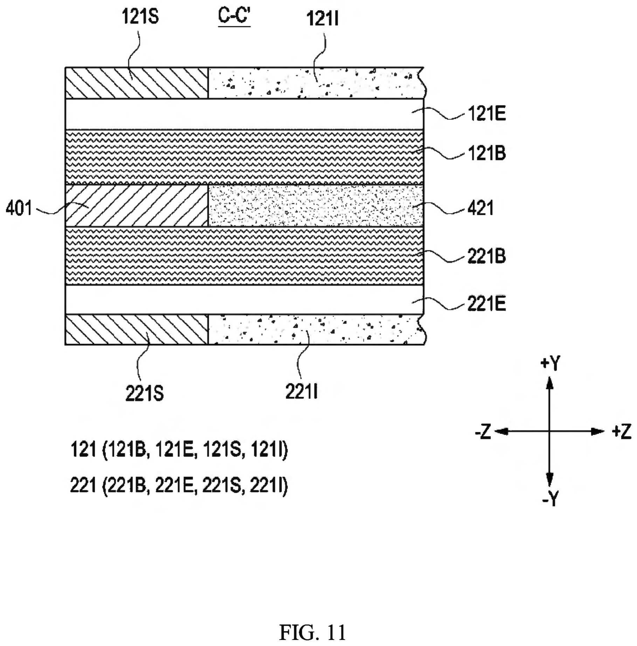

FIG. 4 is a diagram showing a sensor member as a whole according to various embodiments. FIG. 5 is a diagram showing a first sensor and a second sensor. FIG. 6 is a diagram showing a sensor member from another angle according to various embodiments. FIG. 7 is a diagram showing a bent sensor member according to various embodiments. FIG. 8 is a diagram showing area A of FIG. 6. FIG. 9 is a cross-sectional view of section B-B' of FIG. 6. FIG. 10 is a cross-sectional view of section A-A' of FIG. 8. FIG. 11 is a cross-sectional view of section C-C' of FIG. 8.

Referring to FIGS. 4 to 11, the sensor member (10) may include a first sensor (100) and a second sensor (200). The sensor member (10) of FIGS. 4 to 11 may mean a configuration that is entirely or partially the same as the sensor member (10) of FIGS. 1 to 3.

According to various embodiments, the first sensor (100) and the second sensor (200) may be coupled. In one embodiment, the first sensor (100) and the second sensor (200) may be coupled to each other through an adhesive member (421). For example, with respect to the vertical direction (e.g., -y axis direction), an adhesive member (421) may be disposed between the first sensor (100) and the second sensor (200). Here, the adhesive member (421) may be a solid adhesive or a liquid adhesive. In some embodiments, the adhesive member (421) may be provided with an elastic material. In other words, the first sensor (100), the adhesive member (421), and the second sensor (200) may be sequentially stacked.

According to various embodiments, the sensor member (10) can measure at least two or more analytes. In one embodiment, the first sensor (100) may measure a first analyte, and the second sensor (200) may measure a second analyte. Here, the first analyte and the second analyte may include different components. For example, the first sensor (100) may measure glucose, and the second sensor (200) may measure ketone. In addition to glucose and ketone, the sensor member (10) of the present invention may be configured to measure analytes including more various components, but for convenience of description, the following description will focus on the sensor member (10) for measuring glucose and ketone.

According to various embodiments, the sensor member (10) may include a body (301) and a probe (321) extending from the body (301). The body (301) includes a first body (101) of the first sensor (100) and a second body (201) of the second sensor (200), and the probe (321) may include a first probe (121) of the first sensor (100) and a second probe (221) of the second sensor (200).

According to various embodiments, the body (301) is mounted in the above-described body attachable unit (1001) and may be electrically connected to a circuit board (not shown) in the body attachable unit (1001). The probe (321) extends from the body (301) and may be inserted into the body. An electrical signal generated when the probe (321) is inserted into the body and contacts substances in the body may be transmitted to the circuit board through the body (301). For this purpose, the probe (321) and the body (301) may be electrically connected. Here, the substance in the body contacted by the probe (321) may mean interstitial fluid.

According to various embodiments, the first sensor (100) may include all or part of a first body (101), a first link (131), and a first probe (121). According to one embodiment, the first body (101) may be provided in a planar shape. A first contact (103) may be provided on and/or inside the first body (101). For example, the first contact (103) may be an area for electrical connection with the body attachable unit (1001).

According to one embodiment, the first probe (121) may extend from the first body (101) in a first direction (-z axis direction). For example, the first probe (121) may be provided as at least a partial area of the sensor member (10) for insertion into the body. The first probe (121) may be inserted into the body corresponding to the operation of the above-described applicator (e.g., applicator (5) of FIG. 2). In one embodiment, the first probe (121) inserted into the body is contacted with substances in the body (e.g., interstitial fluid) and can measure analytes.

In one embodiment (referring to FIGS. 10 and 11 together), the first sensor (100) may include a first substrate (121B), a first electrode (121E) disposed on the first substrate (121B), a first sensing layer (121S) disposed on the first electrode (121E), and a first insulating layer (121I). For example, on a surface of the first sensor (100) (e.g., +y axis direction surface), the first electrode (121E) and the first sensing layer (121S) may be sequentially disposed. In addition, a first insulating layer (121I) may be disposed adjacent to the first sensing layer (121S). In one embodiment, the first electrode (121E) may extend from the first body (101) to the first probe (121). Alternatively, a pattern for electrical connection may be provided between an edge region of the first probe (121) (e.g., -z axis direction edge) and the first contact (103). For example, the first electrode (121E) may be a carbon electrode, but is not limited thereto. In one embodiment, a first sensing layer (121S) may be disposed in an edge region of the first probe (121) in a first direction (e.g., -z axis direction). For example, the first sensing layer (121S) may include an enzyme for measuring the concentration of glucose and/or ketone. In addition, a membrane may be provided in the first direction edge region of the first probe (121) to cover the first electrode (121E) and/or the first sensing layer (121S).

In some embodiments, the first sensing layer (121S) and the second sensing layer (221S) may be disposed side by side. For example, the first sensing layer (121S) and the second sensing layer (221S) may be disposed side by side along the longitudinal direction (z-axis direction) of the first probe (121). In this case, since the first sensing layer and the second sensing layer are not partitioned by the substrate (first substrate (121B) and/or second substrate (221B)), separate means for partitioning between the first sensing layer and the second sensing layer may be provided.

In FIG. 11, the first sensing layer (121S) is shown as being disposed at one end (-z axis direction end) of the first probe (121), but the present disclosure is not limited thereto. For example, the first sensing layer (121S) may be disposed spaced apart from one end (-z axis direction end) of the first probe (121) by a predetermined distance. In this case, a first insulating layer (1211) may be disposed at one end (-z axis direction end) of the first probe (121), and the first sensing layer (121S) may be surrounded by the first insulating layer (1211). It will be understood that the same description as the first sensing layer (121S) may also apply to the second sensing layer (221S).

According to various embodiments, the second sensor (200) may include all or part of a second body (201), a second link (231), and a second probe (221). According to one embodiment, the second body (201) may be provided in a planar shape. A second contact (203) may be provided on and/or inside the second body (201). For example, the second contact (203) may be an area for electrical connection with the body attachable unit (1001).

According to one embodiment, the second probe (221) may extend from the second body (201) in a first direction (-z axis direction). For example, the second probe (221) may be provided as at least a partial area of the sensor member (10) for insertion into the body. The second probe (221) may be inserted into the body corresponding to the operation of the above-described applicator (e.g., applicator (5) of FIG. 2). In one embodiment, the second probe (221) inserted into the body is contacted with analysis substances in the body (e.g., interstitial fluid) and can measure analytes.

According to various embodiments (referring to FIGS. 10 and 11 together), similar to the first sensor (100), the second sensor (200) may include a second substrate (221B), a second electrode (221E) disposed on the second substrate (221B), a second sensing layer (221S) disposed on the second electrode (221E), and a second insulating layer (221I). For example, on a surface of the second sensor (200) (e.g., +y axis direction surface), the second electrode (221E) and the second sensing layer (221S) may be sequentially disposed. The second electrode (221E) may extend from the second body (201) to the second probe (221). Alternatively, a pattern for electrical connection may be provided between an edge region of the second probe (221) (e.g., -z axis direction edge) and the second contact (203). For example, the second electrode (221E) may be a carbon electrode, but is not limited thereto. In one embodiment, a second sensing layer (221S) may be disposed in an edge region of the second probe (221) in a first direction (e.g., -z axis direction). For example, the second sensing layer may include an enzyme for measuring the concentration of glucose and/or ketone. If the first sensing layer (121S) includes an enzyme for measuring glucose, the second sensing layer (221S) may include an enzyme for measuring ketone. In addition, a membrane may be provided in the second direction edge region of the second probe (221) to cover the second electrode (221E) and/or the second sensing layer (221S). For example, when the first sensing layer (121S) contacts analysis substances in the body, a potential difference occurs between the counter electrode (931) and the first electrode (121E) (and/or the first sensing layer (121S)), which can be used to measure a first component included in the analysis substances. As another example, when the second sensing layer (221S) contacts analysis substances in the body, a potential difference occurs between the counter electrode (931) and the second electrode (221E) (and/or the second sensing layer (221S)), which can be used to measure a second component included in the analysis substances. In this way, by having two or more electrodes (911, 921) share one counter electrode (931), two or more analytes can be measured with one counter electrode (931). In some embodiments, a first potential difference between the first electrode (121E) and the counter electrode (931) and/or a second potential difference between the second electrode (221E) and the counter electrode (931) may be caused simultaneously. Information related to these first potential difference and second potential difference is transmitted to the body attachable unit (1000), and the body attachable unit (1000) can detect the first component and the second component based on the first potential difference and the second potential difference.

According to various embodiments (mainly referring to FIGS. 6 and 8), a counter electrode (401) may be disposed between the first sensor (100) and the second sensor (200) with respect to the vertical direction (e.g., y-axis direction). For example, the counter electrode (401) may be a silver or silver chloride electrode, but is not limited thereto and various counter electrodes performing similar functions may be used. Referring to FIGS. 10 and 11, the above-described first electrode (121E) and/or second electrode (221E) may be provided as working electrodes. In summary, it may be expressed that the first sensor (100) and the second sensor (200) may be substantially symmetrically disposed with the counter electrode (401) and/or the adhesive member (421) therebetween. In addition, the first sensor (100) and the second sensor (200) are partitioned by the counter electrode (401) and/or the adhesive member (421), so that they can measure different analytes.

According to various embodiments, as shown in FIGS. 4, 5, and 7, the sensor member (10) may include a link (331) between the body (301) and the probe (321). The link (331) may be provided by a combination of the first link (131) of the first sensor (100) and the second link (231) of the second sensor (200). In one embodiment, a first neck (132) may be disposed between the first body (101) and the first link (131), and a second neck (232) may be disposed between the second body (201) and the second link (231). According to one embodiment, when the first sensor (100) and the second sensor (200) are overlapped, the first link (131) and the second link (231) may overlap, and the first neck (132) and the second neck (232) may not overlap each other. As shown in FIG. 7, in a state where the first sensor (100) and the second sensor (200) are combined, a bending area (B) near the boundary between the probe (321) and the link (331) may be bent. At this time, since the first neck (132) and the second neck (232) are spaced apart from each other with respect to the horizontal direction (x-axis direction), misalignment of the bonded first sensor (100) and second sensor (200) can be prevented even when the bending area (B) is bent.

In some embodiments, the sensor member (10) may include an overlapping region (O) as shown in FIG. 9. For example, the overlapping region (O) may mean a region where the first sensor (100) and the second sensor (200) overlap each other when the body (301) of the sensor member (10) shown in FIG. 6 is viewed from a direction parallel to the y-axis. In one embodiment, at least a part of the first contact (103) and at least a part of the second contact (203) may not overlap each other in order to provide via holes and/or be electrically connected to a circuit board. Therefore, a partial region (109) of the first body (101) where the first contact (103) located at the first outer side (e.g., -x axis direction) is disposed and a partial region (209) of the second body (201) where the second contact (203) located at the second outer side (e.g., +x axis direction) is disposed may not overlap each other with respect to the y-axis direction. In other words, the first body (101) may include a first extension portion (109) extending from the overlapping region (O) where the adhesive member (421) and the second body (201) are overlapped with respect to the y-axis direction to the first outer side, and the second body (201) may include a second extension portion (209) extending from the overlapping region (O) to the second outer side. In addition, a common contact (305, see FIG. 4) may be disposed in the overlapping region (O). The common contact (305) may be provided by a combination of a first common contact (105, see FIG. 4) of the first sensor (100) and a second common contact (205, see FIG. 4) of the second sensor (200). The first common contact (105) may be at least a part of the first contact (103), and the second common contact (205) may mean at least a part of the second contact (203). The first common contact (105) and the second common contact (205) may be disposed to overlap each other with respect to the vertical direction (y-axis direction). In addition, a via hole may be provided so that the first common contact (105) and the second common contact (205) are connected. In some embodiments, the common contact (305) may be electrically connected to the counter electrode (401).

In some embodiments, the shapes of the first link (131) and the second link (231) may be different from each other. For example, as described above, in order for the first neck (132) and the second neck (232) to be spaced apart from each other when the first sensor (100) and the second sensor (200) are combined, the first neck (132) extends in the -z axis direction from substantially the center of the first link (131) (between the first side (131a) and the second side (131b)), and the second neck (232) may extend while being connected to the first side (231a) of the second link (231). However, it will be understood that this is exemplary and various implementation modifications are possible. For example, the second neck (232) may extend adjacent to the fourth side (231b).

According to various embodiments, the vertical direction (y-axis direction) thickness of the counter electrode (401) may substantially correspond to the vertical direction (y-axis direction) thickness of the adhesive member (421). Thereby, when the first sensor (100) and the second sensor (200) are bonded, unintended thickness change of the probe (321) due to the thickness difference between the adhesive member (421) and the counter electrode (401) can be prevented. According to one embodiment, the adhesive member (421) may include an insulating material including the same material as the first substrate (912, see FIG. 10) and/or the second substrate (922, see FIG. 10). In addition, the adhesive member (421) may include a polymer material that can be applied thinly to correspond the thickness with the counter electrode (401).

Referring to FIG. 10, the sensor member (10) may be partitioned by the counter electrode (931) and may have a stacked structure including at least two or more substrates (912, 922). In addition, it may be expressed that the sensor member (10) may have two or more electrodes sharing the counter electrode (931).

Referring to the illustrated example, with the counter electrode (931) as the center, the first substrate (121B) and the first electrode (121E) may be sequentially disposed in a first vertical direction (+y axis direction), and the second substrate (221B) and the second electrode (221E) may be sequentially disposed in a second vertical direction (-y axis direction). In one embodiment, a combination of the first substrate (121B), the first electrode (121E), and the counter electrode (931) is provided as a means for measuring a first analyte (e.g., glucose), and a combination of the second substrate (221B), the second electrode (221E), and the counter electrode (931) may be provided as a means for measuring a second analyte (e.g., ketone). The first sensor (100) described above in FIGS. 4 to 9 includes a first substrate (121B) and a first electrode (121E), and the second sensor (200) may include a second substrate (221B) and a second electrode (221E).

In one embodiment, the first substrate (121B) and/or the second substrate (221B) may include an insulating material. For example, the first substrate (121B) and/or the second substrate (221B) may include synthetic resin. Specifically, the first substrate (121B) and/or the second substrate (221B) may be a plastic substrate. However, this is exemplary, and various implementation modifications are possible.

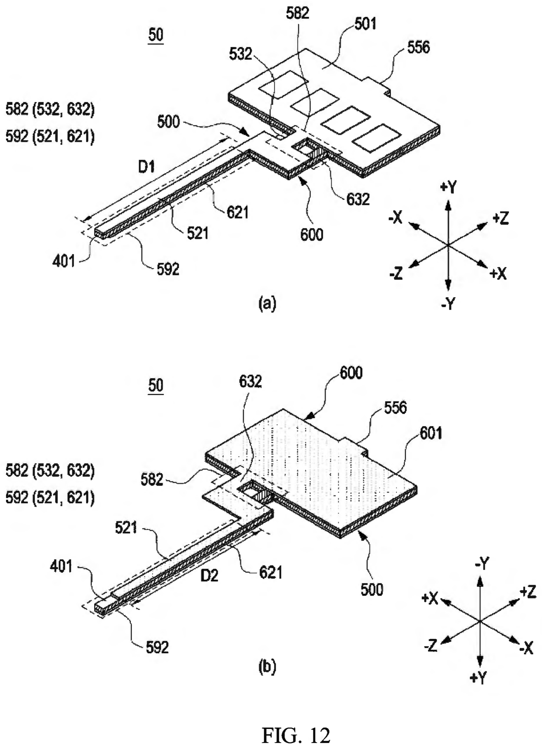

FIG. 12 is a diagram showing a sensor member according to another embodiment. FIG. 12(a) is a perspective view of the sensor member (50), and FIG. 12(b) is a perspective view of the sensor member (50) viewed from a different direction from (a).

Referring to FIG. 12, the counter electrode (401) may be exposed in at least a partial region of the sensor member (50). For the sensor member (50) of FIG. 12, the description of the sensor member (10) of FIGS. 4 to 11 may be applied in whole or in part unless otherwise specified.

According to various embodiments, the first direction (e.g., z-axis) lengths of the first sensor (500) and the second sensor (600) may be provided differently. In one embodiment, the first axis (z-axis) direction lengths of the first probe (521) and the second probe (621) may be different from each other. For example, a second probe length (D2) which is the length of the second probe (621) may be shorter than a first probe length (D1) which is the length of the first probe (521). By providing the second probe length (D2) shorter than the first probe length (D1), the counter electrode (401) may be exposed in a part of the edge region of the probe (592) in the first direction (-z axis direction). As will be described later, as the counter electrode (401) is exposed, the number of via holes for electrical connection between the first electrode (e.g., first electrode (121E) of FIG. 10), the counter electrode (401), and the second electrode (e.g., second electrode (221E) of FIG. 10) can be reduced.

In other words, in the sensor member (50), except for the edge regions of the neck (582) and the probe (592), the first sensor (500) and the second sensor (600) may overlap each other with respect to the vertical direction (y-axis direction).

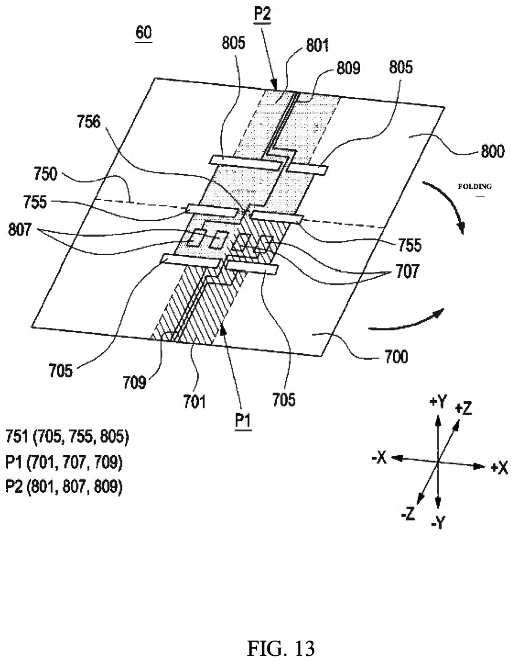

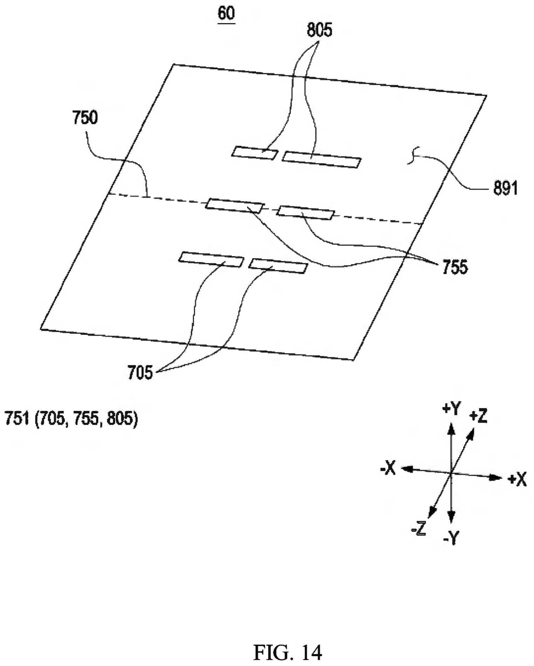

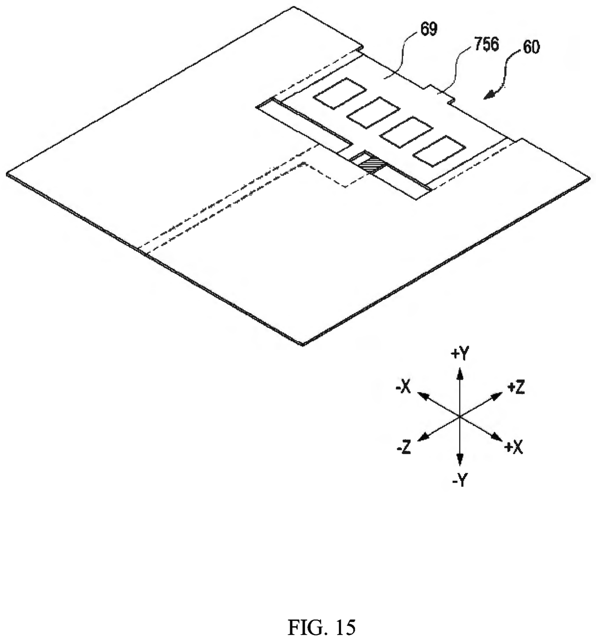

FIGS. 13 to 15 are diagrams showing a manufacturing process of a sensor member according to another embodiment. FIG. 13 is a diagram showing a first surface of a sensor base according to various embodiments. FIG. 14 is a diagram showing a second surface of a sensor base according to various embodiments. FIG. 15 is a diagram showing a folded sensor base according to various embodiments.

Referring to FIGS. 13 to 15, a sensor member including two or more electrodes (e.g., sensor member (50) of FIG. 12) may be provided by folding one substrate (60).

According to various embodiments, the substrate (60) may include a first substrate (700) and a second substrate (800). In one embodiment, the first substrate (700) may be a region corresponding to the above-described first sensor (e.g., first sensor (100) of FIGS. 3 to 11 and/or first sensor (500) of FIG. 12), and the second substrate (800) may be a region corresponding to the above-described second sensor (e.g., second sensor (200) of FIGS. 3 to 11 and/or second sensor (600) of FIG. 12).

According to various embodiments, a folding line (750) may be provided between the first substrate (700) and the second substrate (800). The folding line (750) is provided by processing the surface of the substrate (60) and may allow the substrate (60) to be easily folded.

According to various embodiments, the substrate (60) may include a punched pattern (751). The punched pattern (751) may include all or part of a first punched pattern (705), a second punched pattern (805), and/or a middle punched pattern (755). By providing the punched pattern (751), when the substrate (60) is folded along the folding line (750), the shape of the above-described sensor member (e.g., sensor member (10) of FIG. 4 or sensor member (50) of FIG. 12) can be completed. Here, the folding line (750) may reduce deformation of the substrate (60) when processing the punched pattern (751) on the substrate (60). In one embodiment, a partial region of the substrate (60) between the middle punched patterns (755) may be provided as a connection region (756). The connection region (756) may mean a region where the first substrate (700) and the second substrate (800) are connected. For example, when the substrate (60) is folded to complete a sensor member (e.g., sensor member (50) of FIG. 12), a first sensor (e.g., first sensor (500) of FIG. 12) and a second sensor (e.g., second sensor (600) of FIG. 12) may be connected through a connection region (e.g., connection region (556) of FIG. 12).

According to various embodiments, electrodes may be disposed on a first surface (e.g., a surface facing the +y axis direction) of the substrate (60). For example, a first electrode (701) may be disposed on the first surface of the first substrate (700), and a second electrode (801) may be disposed on the first surface of the second substrate (800).

According to various embodiments, the first substrate (700) may include a first trace (709) for electrical connection with a first contact (707). In addition, the second substrate (800) may include a second trace (809) for electrical connection with a second contact (807).

Referring to FIG. 14, an adhesive member (891) and a counter electrode (841) may be disposed on a second surface (e.g., a surface facing the -y axis direction) of the substrate (60). The counter electrode (841) may be disposed in a region of the second surface, opposing the first electrode (701) and/or the second electrode (801) of the first surface. The adhesive member (891) may be disposed in another region of the second surface where the counter electrode (841) is not disposed.

According to various embodiments, the substrate (60) may be folded along the folding line (750). Thereafter, the sensor member (69) may be separated from the substrate (60) to correspond to the shape of the sensor member (e.g., sensor member (10) of FIG. 4 and/or sensor member (50) of FIG. 12) (see FIG. 15).

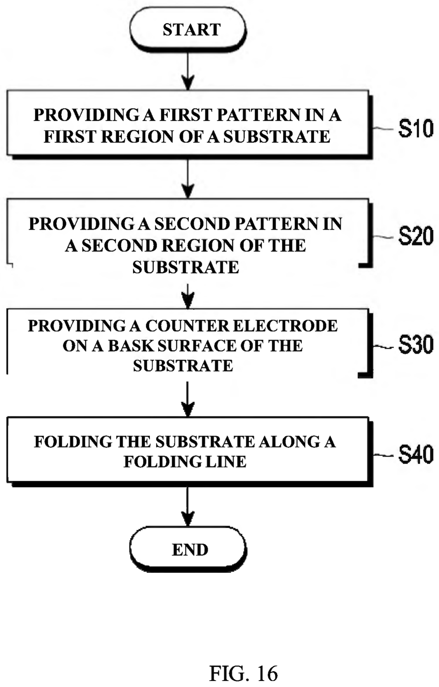

FIG. 16 is a flowchart showing a manufacturing process of a sensor member according to various embodiments.

Referring to FIG. 16, the manufacturing process of the sensor member may include all or part of providing a first pattern in a first region of a substrate (S10), providing a second pattern in a second region of the substrate (S20), providing a counter electrode on a back surface of the substrate (S30), and folding the substrate along a folding region (S40). In describing FIG. 16, the reference numerals described in FIGS. 12 to 15 may be applied together.

According to various embodiments, a first pattern (P1) may be provided in at least one region of a first region of the substrate (S10). Here, the first region may mean a region corresponding to the first sensor (500) on the first surface of the substrate (60) (the surface located in the +Y axis direction in FIG. 13). In addition, the first pattern (P1) may include a first electrode (701), a first contact (707), and a first trace (709) connecting between the first contact (707) and the first electrode (701).

Similarly, a second pattern (P2) may be provided in a second region of the substrate (S20). Here, the second region may mean a region corresponding to the second sensor (600) on the first surface of the substrate (60) (the surface located in the +Y axis direction in FIG. 13). In addition, the second pattern (P2) may include a second electrode (801), a second contact (807), and a second trace (809) connecting between the second contact (807) and the second electrode (801).

According to various embodiments, a counter electrode (841) may be provided on the second surface of the substrate (60). The counter electrode (841) may be provided on a portion of the substrate (60) corresponding to probes (e.g., first probe (121) and/or second probe (221) of FIG. 5). In addition, the adhesive member (891) may be disposed on the second surface of the substrate (60) where the counter electrode (841) is not disposed. Here, as described above, the counter electrode (841) and the adhesive member (891) may be disposed to have substantially the same height.

According to various embodiments, the substrate (60) on which the first pattern (P1), the second pattern (P2), and the counter electrode are disposed may be folded along the folding line (750). By folding the substrate (60), the first substrate (700) and the second substrate (800) are stacked, and the appearance of the substrate (60) may be processed to correspond to the shapes of the various sensor members described above (e.g., sensor member (10) of FIG. 4).

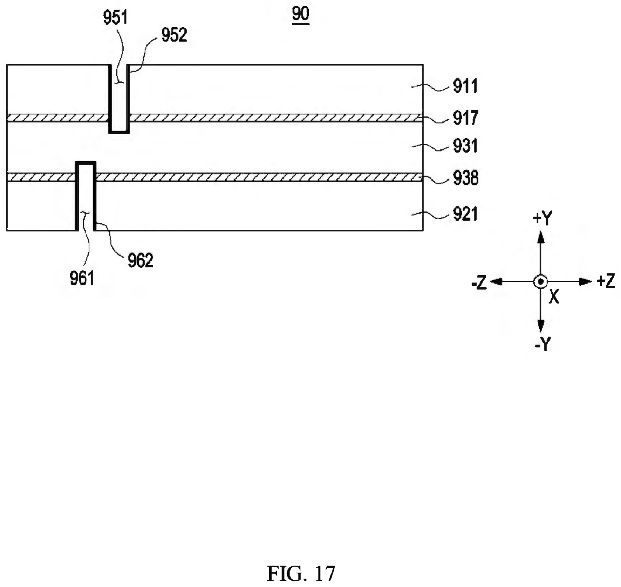

FIG. 17 is a diagram showing a via hole structure according to various embodiments.

Referring to FIG. 17, electrodes (e.g., first electrode (121E) or second electrode (221E)) and the counter electrode (931) may be electrically connected through via holes. The via hole structure shown in FIG. 17 may be provided in the body (301) and/or probe (321) of a sensor member (e.g., sensor member (10) of FIG. 4).

In one embodiment, the first electrode (121E) may be electrically connected to the counter electrode (931) through a via hole, and/or the second electrode (221E) may be electrically connected to the counter electrode (931) through a via hole. The via hole structure of FIG. 17 may be applied to the sensor members in the above-described embodiments (e.g., sensor member (10) of FIG. 4, sensor member (50) of FIG. 11).

According to various embodiments, the first electrode (121E) and the counter electrode (931) may be connected through a first via hole (951). The first via hole (951) may be formed by penetrating through all or partial regions of the first electrode (121E), the first substrate (917), and the counter electrode (931). A first conductive material (952) is applied around the first via hole (951), and through this, the first electrode (121E) and the counter electrode (931) may be electrically connected. In addition, the second electrode (221E) and the counter electrode (931) may be connected through a second via hole (961). The second via hole (961) may be formed by penetrating through all or partial regions of the second electrode (221E), the second substrate (938), and the counter electrode (931). Similarly, the second electrode (221E) and the counter electrode (931) may be electrically connected through a first conductive material (962) provided around the second via hole (961).

According to various embodiments, when viewed from above the sensor member (90) (when viewed parallel to the y-axis), the first via hole (951) and the second via hole (961) may be disposed so as not to overlap each other. In other words, the first via hole (951) and the second via hole (961) may be disposed spaced apart from each other with respect to the longitudinal direction (z-axis direction). Through this, electrical interference between the first via hole (951) and the second via hole (961) can be prevented.

According to various embodiments, the depths of the first via hole (951) and/or the second via hole (961) may be set variously. For example, the first via hole (951) may extend to the inside of the counter electrode (931). As another example, the first via hole (951) may extend to a surface (+y axis direction surface) of the counter electrode (931). At this time, the first conductive material (952) may be applied on the surface of the counter electrode (931). Similar to the first via hole (951), the second via hole (961) may extend to the inside of the counter electrode (931) or may extend to a surface (-y axis direction surface) of the counter electrode (931).

According to various embodiments, a composite sensor may be provided comprising: a first sensor (e.g., first sensor (100) of FIG. 5) including a first substrate (e.g., first substrate (121B) of FIG. 10) comprising a first body (e.g., first body (101) of FIG. 4) and a first probe (e.g., first probe (121) of FIG. 4) extending from the first body, a first electrode (e.g., first electrode (121E) of FIG. 10) disposed on a surface located in a first direction of the first substrate, and a first sensing layer (e.g., first sensing layer (121S) of FIG. 10) electrically connected to the first electrode and disposed on the first probe; a second sensor (e.g., second sensor (200) of FIG. 5) including a second substrate (e.g., second substrate (221B) of FIG. 10) comprising a second body (e.g., second body (201) of FIG. 4) and a second probe (e.g., second probe (221) of FIG. 5) disposed in a region extending from the second body, a second electrode (e.g., second electrode (221E) of FIG. 10) disposed on a surface located in a second direction opposite to the first direction of the second substrate, and a second sensing layer (e.g., second sensing layer (221S) of FIG. 10) electrically connected to the second electrode and disposed on the second probe; and a counter electrode (e.g., counter electrode (931) of FIG. 10) disposed between the first sensor and the second sensor; wherein, when the first sensing layer contacts an analyte in a body, the counter electrode is configured to cause a first potential difference with the first electrode based on a first component included in the analyte, and when the second sensing layer contacts the analyte, the counter electrode is configured to cause a second potential difference with the second electrode based on a second component included in the analyte.

According to one embodiment, the composite sensor may be provided to be electrically connectable to an external device in order to detect the first component and the second component based on the first potential difference and the second potential difference.

According to one embodiment, the composite sensor may be provided to further include a via hole (e.g., first via hole (951) of FIG. 17) for electrical connection between the first electrode and the counter electrode or between the second electrode and the counter electrode.

According to one embodiment, the composite sensor may be provided such that the via hole includes a first via hole connecting the first electrode and the counter electrode and a second via hole connecting the second electrode and the counter electrode.

According to one embodiment, the composite sensor may be provided such that the first via hole and the second via hole are disposed spaced apart so as not to overlap each other when viewed from above the composite sensor.

According to one embodiment, the composite sensor may be provided such that the length of the first probe is shorter than the length of the second probe so that a portion of the counter electrode is exposed to the outside of the composite sensor.

According to one embodiment, the composite sensor may be provided such that the first sensor includes a first link (e.g., first link (132) of FIG. 5) disposed between the first body and the first probe, and the second sensor includes a second link (e.g., second link (232) of FIG. 5) disposed between the second body and the second probe.

According to one embodiment, the composite sensor may be provided such that the first sensor includes a first neck (e.g., first neck (133) of FIG. 5) for connecting between the first link and the first body, the second sensor includes a second neck (e.g., second neck (233) of FIG. 5) for connecting between the second link and the second body, and when viewed from above the composite sensor, the first neck and the second neck are disposed spaced apart.

According to one embodiment, the composite sensor may be provided such that the counter electrode is disposed between an edge of the first probe and an edge of the second probe, an adhesive member (e.g., adhesive member (421) of FIG. 8) is disposed parallel with the counter electrode between the first substrate and the second substrate in the second direction, and the adhesive member and the counter electrode are disposed to have substantially the same thickness.

According to one embodiment, the composite sensor may be provided such that the adhesive member is provided as an insulating material including the same material as the first sensor or the second sensor.

According to one embodiment, the composite sensor may be provided such that the first substrate and the second substrate are connected through a connection portion (e.g., connection portion (556) of FIG. 11).

According to one embodiment, the composite sensor may be provided such that the first component includes glucose and the second component includes ketone.

According to one embodiment, the composite sensor may be provided such that the first probe and the second probe are bent to face a different direction from the first body and the second body.

According to one embodiment, the composite sensor may be provided to include an overlapping region (e.g., overlapping region (O) of FIG. 9) where at least a portion of the first body and at least a portion of the second body are overlapped when viewed from above the composite sensor.

According to one embodiment, the composite sensor may be provided such that the first body includes a first extension portion (e.g., first extension portion (109) of FIG. 9) extending from the overlapping region in a third direction and not overlapping with the second body, and the second body includes a second extension portion (e.g., second extension portion (209) of FIG. 9) extending from the overlapping region in a direction opposite to the third direction and not overlapping with the first body.

According to one embodiment, the composite sensor may be provided such that the first contact and the second contact are provided within the overlapping region.

According to one embodiment, the composite sensor may be provided such that at least a portion of the first contact is disposed in the first extension portion and at least a portion of the second contact is disposed in the second extension portion.

According to various embodiments, a manufacturing process of a composite sensor may be provided to include: disposing electrodes on a first surface of a substrate including a first region and a second region, wherein a first electrode including a first sensing layer for measuring a first component by contacting interstitial fluid in the body is disposed in the first region, and a second electrode including a second sensing layer for measuring a second component different from the first component by contacting the interstitial fluid in the body is disposed in the second region, and disposing a counter electrode on a second surface of the substrate opposite to the first surface; disposing an adhesive member on the second surface of the substrate so as not to overlap with the counter electrode; and folding the substrate along a folding line provided between the first region and the second region.

According to one embodiment, the manufacturing process of the composite sensor may be provided to further include forming a punched pattern (e.g., first punched pattern (705) of FIG. 12) on the substrate to provide an external appearance of the composite sensor.

According to one embodiment, the manufacturing process of the composite sensor may be provided such that the counter electrode and the adhesive member are disposed to have substantially the same height.

Although the embodiments have been described by the limited embodiments and drawings as described above, various modifications and variations are possible from the above description by those skilled in the art. For example, even if the described techniques are performed in a different order from the described method, and/or the components of the described system, structure, device, circuit, etc. are combined or combined in a different form from the described method, or are replaced or substituted by other components or equivalents, appropriate results can be achieved.

Therefore, other implementations, other embodiments, and equivalents to the claims also fall within the scope of the claims described below.

3 terminal
5 applicator
6 button
8 protective cap
10, 50 sensor member
1000 body attachable unit
1001 housing
1050 guide needle
1051 needle head
100, 500 first sensor
200, 600 second sensor
421 adhesive member
301 body
303 contact
305 common contact
321 probe
331 link
332 neck
101 first body
103 first contact
105 first common contact
109 first extension portion
201 second body
203 second contact
205 second common contact
209 second extension portion
121 first probe
131 first link
132 first neck
221 second probe
231 second link
232 second neck
121E first electrode
first substrate
121S first sensing layer
1211 first insulating layer
221E second electrode
221B second substrate
221S second sensing layer
221I second insulating layer
401, 931 counter electrode
60 substrate
751 punched pattern
705 first punched pattern
805 second punched pattern
middle punched pattern
750 folding line
556, 756 connection portion
709 first trace
809 second trace
891 adhesive member
951 first via hole
952 first conductive material
961 second via hole
962 second conductive material

## Claims

1. A composite sensor for measuring at least two analytes, comprising:
a first sensor comprising a first substrate including a first body and a first probe extending from the first body, a first electrode disposed on a surface located in a first direction of the first substrate, and a first sensing layer electrically connected to the first electrode and disposed on the first probe;
a second sensor comprising a second substrate including a second body and a second probe disposed in a region extending from the second body, a second electrode disposed on a surface located in a second direction opposite to the first direction of the second substrate, and a second sensing layer electrically connected to the second electrode and disposed on the second probe; and
a counter electrode disposed between the first sensor and the second sensor;
wherein, when the first sensing layer contacts an analyte in a body, the counter electrode is configured to cause a first potential difference with the first electrode based on a first component included in the analyte, and when the second sensing layer contacts the analyte, the counter electrode is configured to cause a second potential difference with the second electrode based on a second component included in the analyte.

2. The composite sensor of claim 1, wherein the composite sensor is electrically connectable to an external device to detect the first component and the second component based on the first potential difference and the second potential difference.

3. The composite sensor of claim 1, further comprising a via hole for electrical connection between the first electrode and the counter electrode or between the second electrode and the counter electrode.

4. The composite sensor of claim 3, wherein the via hole includes a first via hole extending through the first substrate from the first electrode to the counter electrode and a second via hole extending through the second substrate from the second electrode to the counter electrode.

5. The composite sensor of claim 4, wherein the first via hole and the second via hole are spaced apart so as not to overlap each other when the composite sensor is viewed parallel to the first direction.

6. The composite sensor of claim 4, wherein the composite sensor includes a first conductive material provided on an inner wall of the first via hole and a second conductive material provided on an inner wall of the second via hole.

7. The composite sensor of claim 1, wherein a length of the first probe is shorter than a length of the second probe such that a portion of the counter electrode is exposed to the outside of the composite sensor.

8. The composite sensor of claim 1, wherein the first sensor includes a first link disposed between the first body and the first probe, and the second sensor includes a second link disposed between the second body and the second probe.

9. The composite sensor of claim 8, wherein the first sensor includes a first neck for connecting between the first link and the first body, the second sensor includes a second neck for connecting between the second link and the second body, and when viewed from above the composite sensor, the first neck and the second neck are spaced apart.

10. The composite sensor of claim 1, wherein the counter electrode is disposed between one end of the first probe and one end of the second probe, an adhesive member is disposed parallel with the counter electrode between the first substrate and the second substrate in the second direction, and the adhesive member and the counter electrode are disposed to have substantially the same thickness.

11. The composite sensor of claim 10, wherein the adhesive member includes an insulating material containing the same material as that included in the first sensor or the second sensor.

12. The composite sensor of claim 1, wherein the first substrate and the second substrate are connected through a folding portion.

13. The composite sensor of claim 1, wherein the first component includes glucose, and the second component includes ketone.

14. The composite sensor of claim 1, wherein the first probe and the second probe are bent to face a different direction from the first body and the second body.

15. The composite sensor of claim 1, wherein the composite sensor includes an overlapping region where at least a portion of the first body and at least a portion of the second body are overlapped when viewed parallel to the first direction.

16. The composite sensor of claim 15, wherein the first body includes a first extension portion extending from the overlapping region in a third direction and not overlapping with the second body, and the second body includes a second extension portion extending from the overlapping region in a direction opposite to the third direction and not overlapping with the first body.

17. The composite sensor of claim 15, wherein the first sensor includes a first contact for electrical connection with another device, the second sensor includes a second contact for electrical connection with the another device, and at least a portion of the first contact and the second contact are provided within the overlapping region.

18. The composite sensor of claim 16, wherein the first sensor includes a first contact for electrical connection with another device, the second sensor includes a second contact for electrical connection to the another device, at least a portion of the first contact is disposed in the first extension portion, and at least a portion of the second contact is disposed in the second extension portion.

19. A manufacturing process for a composite sensor, comprising:
disposing electrodes on a first surface of a substrate including a first region and a second region, wherein a first electrode including a first sensing layer for measuring a first component by contacting interstitial fluid in the body is disposed in the first region, and a second electrode including a second sensing layer for measuring a second component different from the first component by contacting the interstitial fluid in the body is disposed in the second region;
disposing a counter electrode on a second surface of the substrate opposite to the first surface;
disposing an adhesive member adjacent to the counter electrode on the second surface of the substrate; and
folding the substrate along a folding line provided between the first region and the second region.

20. The manufacturing process of claim 19, further comprising forming a punched pattern on the substrate to provide an external appearance of the composite sensor.

21. The manufacturing process of claim 20, wherein the counter electrode and the adhesive member are disposed to have substantially the same height.
